# FASCICULE DE BREVET EUROPEEN

(11) **EP 2 736 582 B1**
(45) Date de publication et mention de la délivrance du brevet: **06.05.2015**
(21) Numéro de dépôt: 12740952.2
(22) Date de dépôt: 26.07.2012
(51) Int. Cl.: A61M 39/10

(54) **CONNECTEURS À FLUX DIRECT ANTI-GOUTTE ET À VERROUILLAGE SÉCURISÉ**
TROPFFREIE DIREKTFLUSSVERBINDER MIT SICHERER ARRETIERUNG
NON-DRIP, DIRECT-FLOW CONNECTORS WITH SECURE LOCKING

(30) Priorité: 29.07.2011 FR 1156955
(43) Date de publication de la demande: 04.06.2014
(73) Titulaire: Vygon, 95440 Ecouen (FR)
(72) Inventeur: CARREZ, Jean-Luc, 95440 Ecouen (FR); BARRE, Laurent, 95440 Ecouen (FR); PECH, Marie-Hélène, 75010 Paris (FR)
(74) Mandataire: Regimbeau
(86) Numéro de dépôt international: PCT/EP2012/064669
(87) Numéro de publication internationale: WO 2013/017518

(56) Documents cités:
- WO-A1-00/20070
- WO-A1-2006/062912
- WO-A2-2008/052140
- US-A1- 2003 209 681
- US-A1- 2008 183 155

## Description

L'invention concerne un connecteur pour un circuit de fluide, notamment à usage médical.

Il est connu du document EP 0 544 581 un tel connecteur sécurisé comportant un cône d'entrée distal de type « Luer » femelle destiné à être connecté avec un dispositif présentant un connecteur de type « Luer » mâle compatible et complémentaire. Le cône d'entrée distal est obturé par un septum ou membrane présentant une surface affleurante qui est facilement nettoyable pour enlever les germes ou bactéries avant de réaliser une éventuelle connexion. Le septum protège un tube qui, une fois la connexion réalisée, s'étend en partie dans le trou du connecteur de type « Luer » mâle qui lui est connecté. Une fois connecté, le connecteur du document EP 0 544 581 présente un passage tubulaire droit et un volume mort réduit. Le connecteur décrit dans le document EP 0 544 581 est un connecteur sécurisé femelle.

On a ensuite proposé dans la demande internationale PCT/EP 2011/052315 un ensemble de connexion sécurisée pour un circuit liquide, comprenant un connecteur mâle et un connecteur femelle, munis chacun d'un raccord proximal et d'un raccord distal. Le raccord distal de chacun des connecteurs définit un passage et comprend une partie tubulaire dans laquelle s'étend de manière coaxiale un organe de connexion monté fixe sur le raccord proximal, et une membrane déformable élastiquement, sensiblement tubulaire, fermée à une extrémité distale par une épaisseur de membrane. La membrane de chaque connecteur est mobile entre une position d'obturation, dans laquelle elle recouvre une extrémité libre de l'organe de connexion de manière étanche, et une position de connexion, dans laquelle elle est traversée par l'organe de connexion. Par ailleurs, la partie tubulaire du raccord distal du connecteur mâle est adaptée pour être insérée, lors d'une connexion, dans la partie tubulaire du raccord distal du connecteur femelle, de sorte que, en position d'obturation, pour chacun des connecteurs mâle et femelle, la membrane obture de manière étanche le passage du raccord distal.

Cet ensemble de connexion présente l'avantage de réduire le nombre d'étapes nécessaires pour son assemblage par un opérateur. Par ailleurs, il comprend des moyens de connexion facilement nettoyables, notamment au niveau des parties distales des connecteurs, dans la mesure où les membranes affleurent l'extrémité distale des connecteurs.

Par ailleurs, le document WO 2006/062912 décrit un raccord Luer mâle étanche pouvant être fixé à une valve Luer femelle standard en vue de l'ouverture d'un canal d'écoulement intermédiaire. Pour cela, le raccord mâle comprend un compartiment rigide possédant une extrémité distale pourvue d'un raccord Luer mâle rigide et une extrémité proximale au niveau de laquelle un joint proximal est formé. L'extrémité distale du compartiment comprend un siège de valve. Un élément de sollicitation élastique est disposé dans le compartiment, cet élément sollicitant un actionneur en contact avec le siège de valve de manière à empêcher un écoulement fluidique à travers le raccord mâle. Lors d'une connexion avec un raccord femelle, l'actionneur est déplacé dans le sens proximal de façon à ouvrir la valve distale puis le joint proximal. Un vide partiel est formé dans le raccord mâle lors d'une désolidarisation du raccord femelle, afin d'aspirer les liquides sur la surface extérieure de l'extrémité distale du raccord mâle dans la pointe mâle.

Le document WO 00/20070 quant à lui décrit un connecteur point de pénétration sans aiguille comportant un corps pointu possédant une pointe creuse, un Luer femelle, un manchon en pointe souple et élastique pourvu d'une tête et d'une partie formant ressort s'étend au-dessus de la pointe, un élément de centrage recouvrant une partie de la pointe et du manchon et un septum élastique nettoyable placé entre l'élément de centrage et une extrémité du Luer femelle. Le corps pointu et le Luer femelle sont assujettis l'un à l'autre et terminent l'ensemble. La longueur du Luer et/ou la longueur de la pointe sont telles que, lorsqu'un Luer mâle est poussé dans le Luer femelle, il exerce une pression sur le septum et l'applique sur la pointe, de sorte que la tête du manchon se trouve percée par le sommet de la pointe. Lorsque le septum est encore comprimé, il s'étend au-dessus de la pointe, de sorte que la cavité de la pointe est en communication fluidique avec le raccord Luer mâle. Ce point d'injection ne provoque qu'un reflux minime et le septum constitue une double barrière antibactérienne nettoyable au-dessus de la pointe.

Enfin, le document WO 2008/052140 décrit un connecteur médical comprenant un boîtier rigide, un élément interne rigide disposé dans le boîtier et comportant au moins une ouverture latérale, et un élément creux flexible agencé dans le boîtier dont une projection est adaptée pour pénétrer dans l'ouverture latérale.

Il est important pour ces connecteurs médicaux pour circuit liquide de limiter voir supprimer les risques de formation d'une goutte en sortie des connecteurs, au moment de leur séparation. En effet, les produits transférés par l'intermédiaire de ces connecteurs sont généralement des médicaments plus ou moins dilués, qui peuvent donc être dangereux pour les utilisateurs ou les patients si ceux-ci s'y trouvent régulièrement exposés, comme cela peut être le cas pour les préparateurs de médicaments. La formation de gouttes en sortie des connecteurs peut en outre être source de contaminations croisées dans les préparations des médicaments elles-mêmes.

Un objectif de l'invention est donc de proposer un nouvel ensemble de connecteurs mâle et femelle à connexion sécurisée pour un circuit de fluide, dont la membrane et le corps soient parfaitement nettoyables, tout en limitant voire supprimant la formation de gouttes lors de la séparation des connecteurs.

De manière secondaire, un autre objectif de la demande est de proposer un ensemble de connecteurs pour un circuit de fluide qui limite les risques de dégradation des fluides le traversant, tels que les risques de destruction du culot globulaire.

Pour cela, l'invention propose un connecteur mâle pour un circuit liquide, comprenant :
- un raccord distal comprenant une partie tubulaire définissant un passage,
- un raccord proximal,
- un organe de connexion s'étendant de manière fixe et coaxiale dans la partie tubulaire du raccord distal, ledit organe de connexion étant constitué d'un corps tubulaire sur lequel est fixé, au niveau d'une extrémité libre, une valve cylindrique déformable élastiquement et fermée au niveau d'une extrémité distale par une épaisseur de valve traversée par une fente, et
- une bague coulissante montée sur le corps tubulaire, ladite bague coulissante étant mobile entre une position inactive, dans laquelle elle encapsule les parois de la valve en laissant libre l'extrémité distale de ladite valve, et une position de connexion, dans laquelle elle est située à distance de l'extrémité distale de la valve et dévoile tout ou partie des parois latérales de la valve.

Certains aspects préférés mais non limitatifs du connecteur mâle selon l'invention sont les suivants :
- il comprend en outre un moyen de rappel prenant appui entre la bague coulissante et le corps tubulaire, et adapté pour ramener la bague coulissante vers sa position de repos ;
- la valve ferme l'extrémité libre du corps tubulaire de manière étanche ;
- la valve présente en outre au moins une gorge borgne débouchant sur le corps tubulaire, ladite gorge s'étendant de manière sensiblement parallèlement à la fente, coaxialement au corps tubulaire, ou autour de la fente ;
- la valve est surmoulée sur le corps tubulaire ;
- l'extrémité distale de la valve présente une surface concave ;
- en position de repos, une surface distale de l'extrémité distale de la valve affleure une surface distale de l'extrémité distale de la bague coulissante ;
- la valve comprend en outre, au niveau de la fente, une surépaisseur radiale s'étendant coaxialement au corps tubulaire ;
- la surépaisseur a la forme d'une ellipse dont un petit diamètre est sensiblement égal à un diamètre interne de la bague coulissante et un grand diamètre mesure quelques dixièmes de millimètres de plus que le petit diamètre ;
- le raccord distal encapsule le corps tubulaire et la bague coulissante de manière à ne laisser libre que leurs extrémités distales respectives ;
- la bague coulissante comprend en outre un joint d'étanchéité s'étendant entre la bague coulissante et le raccord distal ;
- le joint d'étanchéité est disposé dans une cavité radiale de ladite bague coulissante, ou surmoulé sur ladite bague coulissante ;
- l'extrémité distale du corps tubulaire comprend un épaulement adapté pour recevoir la valve, de sorte qu'une surface externe des parois latérales de la valve et du corps tubulaire est continue ;
- le corps tubulaire définit un canal interne de section sensiblement constante ;
- le raccord distal comprend en outre des moyens de verrouillage ;
- les moyens de verrouillage sont à baïonnettes ;
- une extrémité distale du raccord distal présente des parois de centrage dans le prolongement du raccord distal ;
- le corps tubulaire présente en outre une fente s'étendant parallèlement à la fente de la valve depuis son extrémité distale en direction de son extrémité proximale, de manière à définir deux pattes latérales ; et
- la fente de la valve est biseautée en direction du corps tubulaire.

Selon un deuxième aspect, l'invention propose un procédé de fabrication d'un connecteur mâle conforme à l'invention, comprenant les étapes consistant à :
- prévoir un corps tubulaire ;
- fixer la valve sur une extrémité distale du corps tubulaire ;
- fixer le corps tubulaire sur le raccord proximal;
- monter la bague coulissante sur l'extrémité distale du corps tubulaire ; et
- fixer le raccord distal sur le corps tubulaire.

Certains aspects préférés mais non limitatifs du corps tubulaire conforme à l'invention sont les suivants :
- il comprend en outre une étape au cours de laquelle on réalise la fente dans la valve après fixation de la valve sur l'extrémité distale à du corps tubulaire ; et
- la valve est fixée par surmoulage sur le corps tubulaire.

Selon un troisième aspect, l'invention propose un ensemble de connexion pour un circuit liquide, comprenant un connecteur mâle conforme à l'invention, et un connecteur femelle, ledit connecteur femelle comprenant :
- un raccord distal, et
- un raccord proximal, le raccord distal comprenant une partie tubulaire, dans laquelle s'étend de manière coaxiale un organe de connexion monté fixe sur le raccord proximal, et une membrane cylindrique déformable élastiquement fermée au niveau d'une extrémité distale par un septum traversé par une fente, et mobile entre une position d'obturation, dans laquelle la membrane recouvre une extrémité libre de l'organe de connexion de manière étanche, et une position de connexion, dans laquelle la membrane et la valve sont traversées par l'organe de connexion.

Certains aspects préférés mais non limitatifs de l'ensemble sont les suivants :
- la forme et les dimensions de la valve du connecteur mâle sont sensiblement égales à la forme et aux dimensions de la membrane, de sorte que lors de la connexion du connecteur mâle avec le connecteur femelle, la valve est apte à pénétrer dans l'extrémité distale du raccord distale du connecteur femelle ;
- l'extrémité distale de la membrane présente une surface convexe, tandis que l'extrémité distale de la valve présente une surface concave, la concavité de la surface de l'extrémité distale de la valve correspondant à la convexité de surface de l'extrémité distale de la membrane ;
- le connecteur femelle et le connecteur mâle comprennent chacun des moyens de verrouillage complémentaires à baïonnettes ;
- les dimensions et la forme du raccord distal du connecteur femelle sont sensiblement identiques aux dimensions et à la forme de la bague coulissante, de sorte que lors de la connexion du connecteur mâle avec le connecteur femelle, l'extrémité distale du connecteur femelle soit apte à pénétrer dans l'extrémité distale du raccord distal du connecteur mâle ; et
- le corps tubulaire du connecteur mâle présente en outre une fente s'étendant parallèlement à la fente de la valve depuis son extrémité distale en direction de son extrémité proximale de manière à définir deux pattes latérales, et dans lequel les pattes latérales du connecteur mâle se déforment élastiquement lors de la pénétration de l'organe de connexion du connecteur femelle dans la fente de la valve du connecteur mâle.

Selon un quatrième aspect, l'invention propose l'utilisation d'un ensemble de connecteurs conforme à l'invention, comprenant les étapes consistant à :
- placer l'extrémité distale (120) du connecteur femelle (100) contre l'extrémité distale (10) du connecteur mâle (1), de sorte que la valve (40) soit en contact avec la membrane (130);
- insérer le raccord distal (140) du connecteur femelle (100) dans le raccord distal (10) du connecteur mâle (1), en poussant le connecteur femelle (100) en direction du connecteur mâle (1), de sorte que la valve (40) pénètre dans l'extrémité distale du raccord distal (140) du connecteur femelle (100) et que le raccord distal (10) du connecteur mâle (1) déplace la bague coulissante (50) en direction du raccord proximal du connecteur mâle (1), jusqu'à ce que l'organe de connexion (124) du connecteur femelle (100) traverse à la fois la membrane (130) et la valve (40) ; et
- verrouiller l'ensemble.

D'autres caractéristiques, buts et avantages de la présente invention apparaîtront mieux à la lecture de la description détaillée qui va suivre, et en regard des dessins annexés donnés à titre d'exemples non limitatifs et sur lesquels :
La figure 1 est une vue en perspective d'une forme de réalisation d'un connecteur femelle pouvant être utilisé dans un ensemble conforme à l'invention ;
La figure 2 est une vue en coupe du connecteur femelle de la figure 1 ;
La figure 3 est une vue en perspective d'une forme de réalisation d'un connecteur mâle conforme à l'invention ;
La figure 4 est une vue en coupe du connecteur mâle de la figure 3 ;
La figure 5A est une vue en coupe d'un exemple de réalisation d'un corps tubulaire sur lequel est fixé une valve conforme à l'invention ;
La figure 5B est une vue en perspective de la partie distale de l'exemple de réalisation de la figure 5A ;
La figure 6 est une vue en perspective d'une forme de réalisation de la partie distale d'un corps tubulaire conforme à l'invention ;
La figure 7A est une vue en perspective d'une section d'un exemple de réalisation d'une valve conforme à l'invention ;
La figure 7B est une vue en coupe de la figure 7A ;
La figure 7C est une vue en perspective de la partie proximale d'une valve conforme à la figure 7A ;
La figure 8A est une vue en perspective d'un ensemble comprenant le corps tubulaire et la valve de la figure 5B, un exemple de réalisation d'une bague coulissante et un exemple de réalisation d'un moyen élastique conformes à l'invention ;
La figure 8B est une vue en coupe de la partie distale de l'ensemble de la figure 7 ;
La figure 9 est une vue de côté d'un connecteur femelle et d'un connecteur mâle en contact avant assemblage ;
La figure 10 est une vue de coupe des connecteurs femelle et mâle de la figure 9 ;
La figure 11 est une vue de côté d'un connecteur femelle et d'un connecteur mâle connectés et verrouillés ;
La figure 12 est une vue de coupe des connecteurs femelle et mâle de la figure 11 ;
La figure 13 est une vue en détail de la connexion du connecteur femelle avec le connecteur mâle de la figure 12.

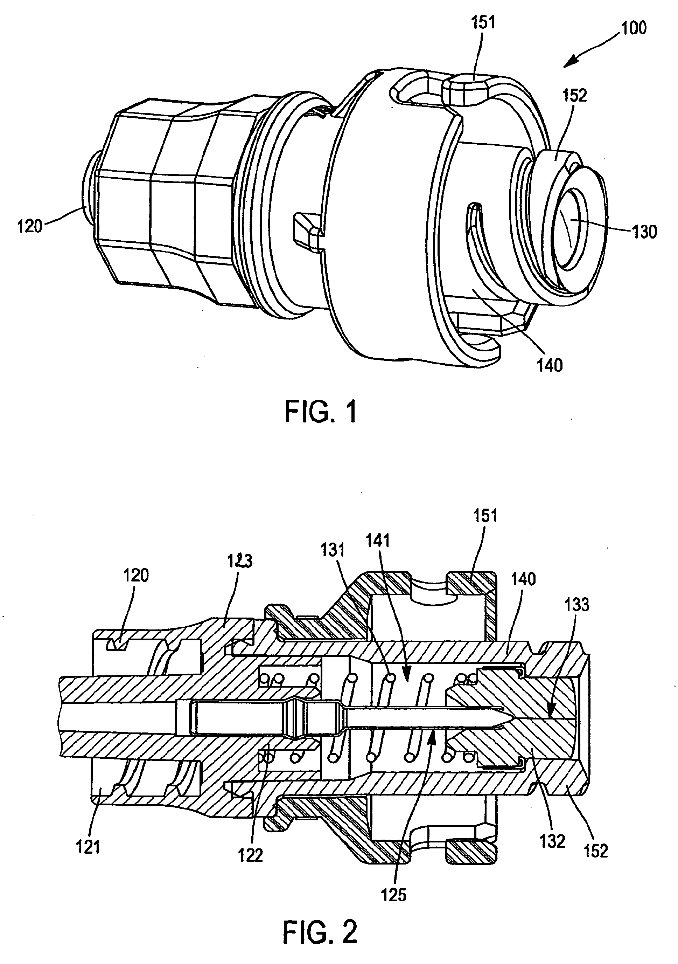

En référence aux figures 1 à 2, nous allons tout d'abord décrire un connecteur femelle 100 selon l'invention.

Le connecteur femelle 100 comporte un raccord proximal 120, une membrane 130 déformable élastiquement, sensiblement tubulaire, et un raccord distal 140 solidaire du raccord proximal 120.

Le raccord proximal 120 est de forme générale tubulaire et comporte une extrémité proximale qui comprend, ici, une entrée connectable 121 de type "Luer-Lock" femelle. En variante de réalisation, cette entrée connectable 121 est de type "Luer-Lock" mâle. Elle se prolonge, en direction distale, par un corps creux 122.

Le corps creux 122 du raccord proximal 120 se termine, en direction distale, par un rebord 123 de forme circulaire surmonté de manière coaxiale par un tube de connexion creux 124 qui s'étend en saillie du raccord proximal 120 en direction distale. Le tube creux 124 se termine par une extrémité libre 125 distale et forme un organe de connexion.

Nous allons maintenant décrire plus en détail la membrane 130 du connecteur 100.

Cette membrane 130 présente une extrémité distale 132, et comporte des moyens élastiques 131.

Ici, les moyens élastiques 131 sont un ressort 131, par exemple du type ressort de compression hélicoïdal, s'étendant jusqu'à l'extrémité distale 132. Ils sont destinés à prendre appui sur le raccord proximal 120 et à venir pousser l'extrémité distale 132 de la membrane 130 de manière à ce que cette dernière vienne dans une position de repos (inactive), dans laquelle elle obture une extrémité distale du passage 141.

L'extrémité distale 132 de la membrane 130 est un septum présentant une surface distale globalement convexe traversée par une fente coaxiale au tube de connexion 124.

Ainsi le connecteur femelle 100 comporte une membrane 130 qui est encapsulée dans la partie tubulaire du raccord distal 140, de sorte que seule la surface distale traversée par la fente soit accessible par un opérateur. Ceci permet, au surplus, en position de repos, un serrage radial de la membrane 130, et en particulier de l'extrémité distale 132 de cette dernière, réalisé par une paroi de la partie tubulaire du raccord distal 140 : une meilleure étanchéité et une tenue en contre-pression améliorée sont alors obtenues.

Sur sa périphérie, de préférence en partie proximale, le raccord distal 140 comporte un moyen de verrouillage 150. Ici, le moyen de verrouillage est femelle et à baïonnettes et comprend une jupette 151 pourvue de gorges adaptées pour coopérer avec des ergots mâles d'un moyen de verrouillage à baïonnettes complémentaire. Dans la forme de réalisation illustrée sur les figures 1 et 2, l'extrémité distale 152 du raccord distal 140, qui est entourée par la jupette 151, est filetée de manière à constituer une entrée connectable de type « Luer-Lock » femelle. Pour cela, la partie distale de la jupette 151 est raccourcie et décalée de sorte que la face l'extrémité distale 151 soit accessible radialement. Ainsi, on obtient un moyen de verrouillage femelle pouvant être connecté soit à des ergots d'un moyen de verrouillage mâle à baïonnettes, soit à un moyen de verrouillage de type "Luer-Lock" mâle complémentaire.

Ces moyens de verrouillage 150 seront détaillés plus loin dans la description.

Une description plus détaillée d'un connecteur similaire au connecteur 100 est fournie dans le document PCT/EP 2011/052315 cité plus haut auquel il est possible de se référer à cette fin.

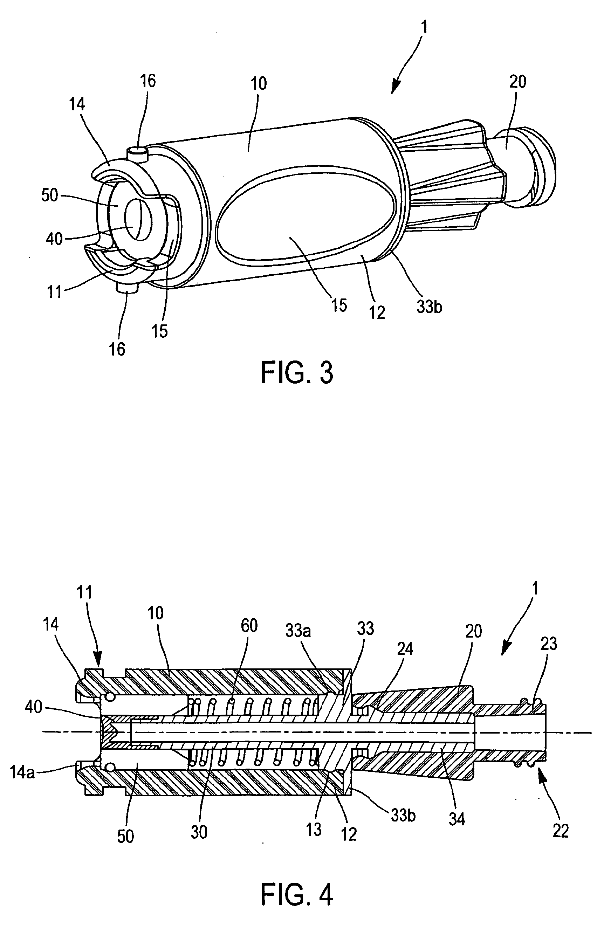

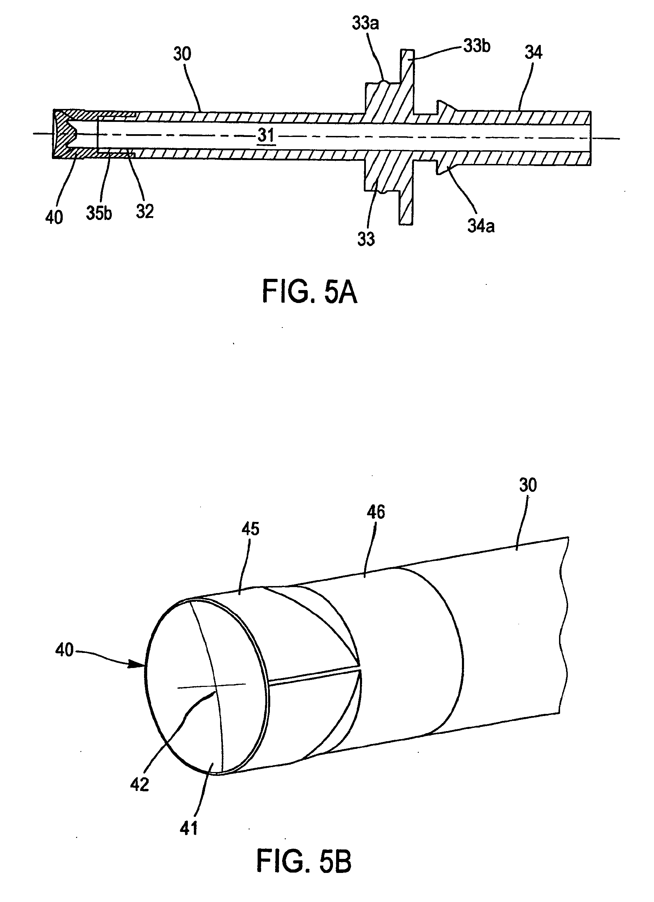

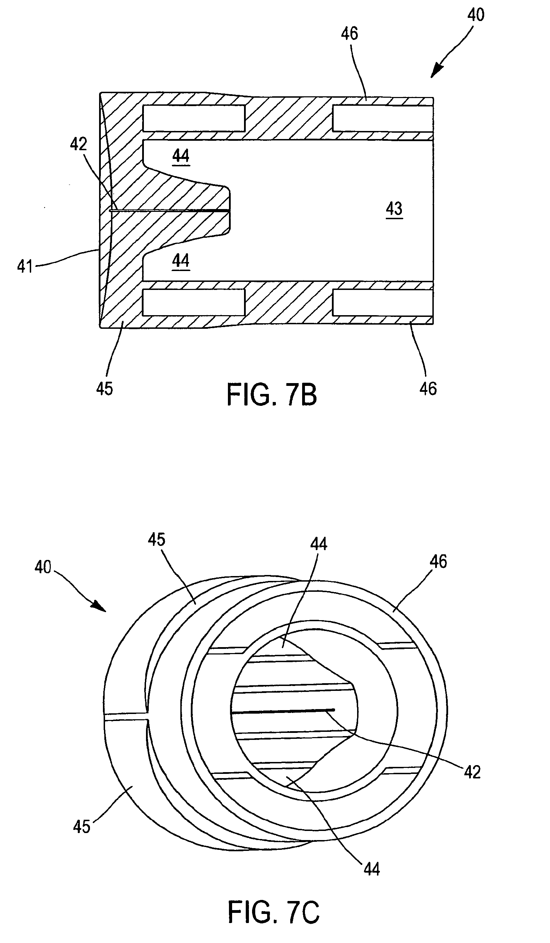

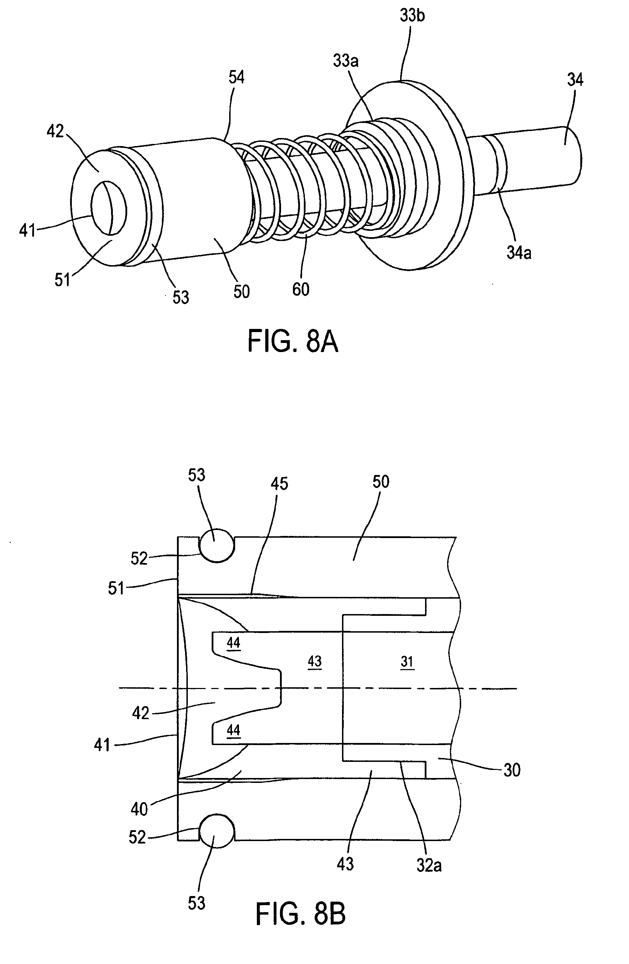

Nous allons à présent décrire un connecteur mâle 1 selon l'invention en référence aux figures 3 à 8B.

Le connecteur mâle 1 comprend un raccord distal 10 comportant une partie tubulaire définissant un passage, un raccord proximal 20 et un organe de connexion 30.

L'organe de connexion 30 s'étend dans le passage 11 du raccord distal 10, coaxialement à la partie tubulaire de celui-ci. Il a la forme d'un corps globalement tubulaire formant un canal interne 31 globalement cylindrique de révolution, et présente une valve 40 au niveau d'une extrémité distale 32.

Le corps tubulaire 30 est inséré dans le raccord distal 10 de telle sorte que la valve 40 soit adjacente à l'extrémité distale 11 du raccord distal.

Il comprend en outre un renflement radial 33 adapté pour venir en prise avec l'extrémité proximale du raccord distal 12. Par exemple, le renflement 33 présente la forme d'un disque s'étendant transversalement à l'axe du corps tubulaire, dont le diamètre externe est sensiblement égal au diamètre interne du raccord distal 10. Le disque présente en outre des moyens d'encliquetage 33a, par exemple une saillie radiale pouvant être annulaire, adaptée pour coopérer avec une gorge 13 de forme complémentaire réalisée dans la face interne du raccord distal 10, et est prolongé par une collerette 33b de plus grand diamètre formant butée pour le raccord distal 10. Ainsi, lorsque le corps tubulaire 30 est inséré dans le raccord distal 10, l'extrémité proximale 12 du raccord distal 10 vient en appui contre la collerette 33b formant butée et vient en prise avec la saillie annulaire 33a, de sorte que le raccord distal 10 se trouve bloqué en translation par rapport au corps tubulaire 30.

Enfin, le corps tubulaire 30 se prolonge en partie proximale au-delà du renflement radial 33 par une zone 34 d'accrochage au raccord proximal 20. La zone d'accrochage 34 comprend un corps cylindrique présentant, au niveau d'une face externe, au moins un organe de connexion 34a. De préférence, la zone d'accrochage 34 est cylindrique de révolution, l'organe de connexion 34a ayant alors la forme d'une protubérance annulaire. De la sorte, le raccord proximal 20 est fixé en translation sur le corps tubulaire 30 mais libre en rotation autour de celui-ci, afin d'éviter des desserrages accidentels du raccord proximal 20 du connecteur mâle 1.

La valve 40 du corps tubulaire 30 est une membrane élastiquement déformable, de forme globalement cylindrique, et fermée au niveau d'une extrémité distale 41 par une membrane. Le diamètre extérieur de la valve 40 est inférieur ou égal au diamètre extérieur de la membrane 130, de préférence sensiblement égal.

L'épaisseur de membrane est traversée par une fente 42 qui débouche sur le corps tubulaire 30. L'extrémité distale du corps tubulaire 32 et la fente 42 peuvent être séparées par un espace interne 43 prolongeant le canal interne 31.

La fente 42 peut être droite ou biseautée en direction du corps tubulaire 30, en fonction notamment de son procédé de fabrication.

Par exemple, la valve 40 est réalisée dans un matériau élastomère permettant de fermer le corps tubulaire 30 de manière étanche malgré la présente de la fente 42. Typiquement, la valve 40 peut être réalisée dans du silicone LSR (acronyme anglais de Liquid Silicon Rubber, pour caoutchouc silicone liquide), tandis que le corps tubulaire 30 est réalisé dans un matériau plastique, tel qu'une résine plastique PBT (Polybutylène Téréphtalate). Elle peut alors être surmoulée sur le corps tubulaire 30. Dans ce cas, afin d'améliorer la liaison entre la valve 40 et le corps tubulaire 30, l'extrémité distale du corps tubulaire 30 peut notamment être traversée radialement par un ou plusieurs orifices 36. Par ailleurs, la fente 42 est de préférence réalisée par découpe à travers l'épaisseur de membrane après fixation de la valve 40 sur le corps tubulaire 30, par exemple à l'aide d'un scalpel, de manière à être indexée avec précision par rapport au corps tubulaire 30.

Avantageusement, l'extrémité distale 32 du corps tubulaire 30, sur laquelle est fixée la valve 40, présente un épaulement annulaire 32a formé par rétrécissement de la paroi du corps tubulaire 30 afin de recevoir la valve 30 sans créer de discontinuité au niveau de la surface externe de l'organe de connexion. La valve 40 est alors surmoulée (ou fixée) sur cet épaulement 32a sur une épaisseur telle que la paroi externe 46 de la valve 40 et le corps tubulaire 30 forment une surface continue et globalement lisse.

Selon une forme de réalisation, la valve 40 comprend en outre au moins une gorge borgne 44 débouchant sur le corps tubulaire 30 et qui est coaxiale au corps tubulaire 30.

La gorge 44 a un effet double sur la fente 42.

Lors de l'ouverture de la fente 42 par introduction d'un objet depuis l'extrémité distale 41 de celle-ci, comme nous le verrons plus bas dans la description, la gorge 44 permet à la fente 42 de s'ouvrir plus facilement, les bords de la fente 42 n'étant pas retenus en raison de l'espace vide créé par la gorge 44.

Par ailleurs, lorsque la fente 42 est fermée (et donc non traversée par un objet tel que le tube de connexion 124), en cas de remontée de fluide en provenance du corps tubulaire 30, le fluide pénètre dans la gorge 44 et applique une pression sur les bords de la fente qui tend à plaquer les bords de la fente 42 l'un vers l'autre. La fente 42 est donc maintenue fermée et empêche le fluide de sortir, augmentant ainsi l'étanchéité du connecteur 1.

La gorge 44 peut par exemple être de forme annulaire et s'étendre autour et à distance de la fente, transversalement à l'axe du corps tubulaire 30. En variante, la valve 40 comprend deux gorges 44 sensiblement rectilignes s'étendant parallèlement à la fente 42, de part et d'autre de celle-ci. Cette forme de réalisation présente l'avantage d'appliquer une pression plus efficace sur la fente 42, et donc d'améliorer sa résistance en contre-pression. Selon une autre variante encore, les gorges 44 sont courbes et de part et d'autre de la fente.

Enfin, selon une forme de réalisation, l'extrémité distale 41 de la valve 40 est une surface concave. La surface de l'extrémité distale de la membrane 130 du connecteur femelle 100 étant de forme convexe, cela permet à l'extrémité distale de la valve 40 d'épouser parfaitement la surface de la membrane 130, et de limiter ainsi la fuite de fluide lorsque les connecteurs 1 et 100 sont connectés ensemble. De préférence, la concavité de l'extrémité distale 41 de la valve 40 correspond à la convexité de l'extrémité distale de la membrane 130.

En variante, la surface de l'extrémité distale de la membrane 130 du connecteur femelle 100 est de forme concave ; l'extrémité distale 41 de la valve 40 est alors une surface convexe de convexité correspondante.

Le connecteur mâle 1 comprend en outre une bague coulissante 50 montée sur le corps tubulaire 30, mobile entre une position inactive dite de repos et une position de connexion.

Dans la position de repos, la bague 50 encapsule les parois latérales 46 de la valve 40, en laissant libre son extrémité distale 41. De préférence, l'extrémité distale 41 de la valve 40 affleure la surface de l'extrémité distale 51 de la bague 50, de manière à minimiser les arêtes et à faciliter le nettoyage du connecteur 1. Cette position est notamment illustrée en figures 3, 4, 8A, 8B et 10.

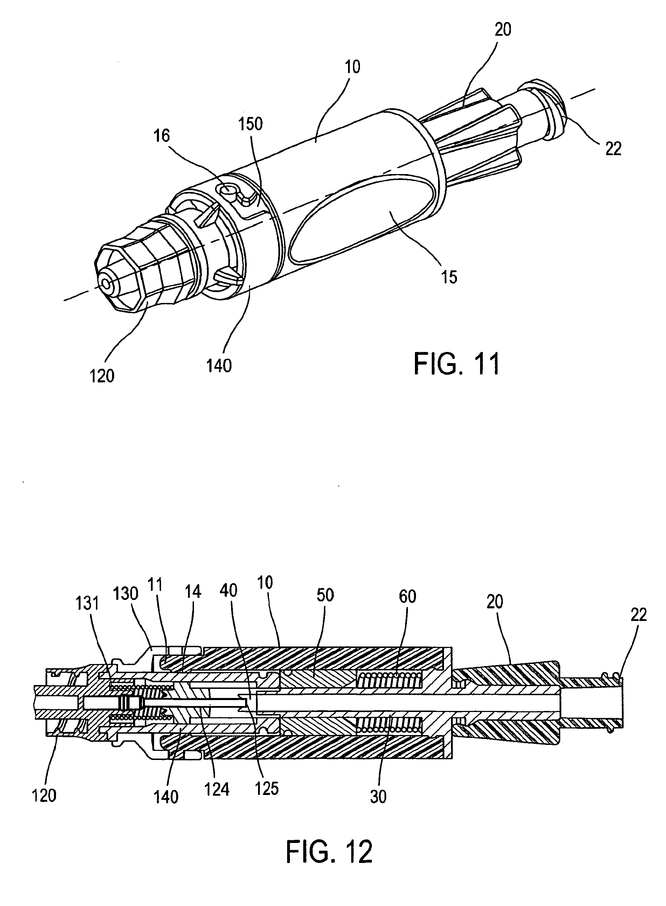

Dans la position de connexion, illustrée notamment sur les figures 11 à 13, la bague 50 est située à distance de l'extrémité distale 41 de la valve 40 et dévoile tout ou partie de la valve 40.

Avantageusement, le diamètre interne de la bague 50 est sensiblement égal au diamètre externe du corps tubulaire 30 et de la valve 40.

La valve 40 peut en outre présenter une légère surépaisseur 45 (de l'ordre de 5% de l'épaisseur totale de la valve 40) s'étendant radialement au niveau de son extrémité distale 41, de manière adjacente à la fente 42. De la sorte, en position de repos, la bague 50, dont le diamètre interne est légèrement plus petit que le diamètre externe de la valve 40 au niveau de la surépaisseur 45, exerce une force radiale centripète sur la valve 40 et la fente 42. Par conséquent, au repos, la bague 50 augmente encore la résistance en contre-pression de la valve 40.

Par ailleurs, la surépaisseur 45 permet d'éviter le passage de fluide entre la bague 50 et la valve 40.

Dans la forme de réalisation illustrée sur les figures, la surépaisseur 45 n'est présente que de chaque côté de la fente 42, afin que la pression ne soit exercée que de part et d'autre de celle-ci, améliorant ainsi sa résistance en contre-pression. Par exemple, la surépaisseur peut avoir la forme d'une ellipse dont le petit diamètre (au niveau des extrémités de la fente 42) est sensiblement égal au diamètre interne de la bague 50 tandis que le grand diamètre (au niveau des bords de la fente 42) mesure quelques dixièmes de millimètres de plus que le petit diamètre.

Le diamètre externe de la bague coulissante 50 est de préférence sensiblement égal au diamètre interne du raccord distal 10, de manière à limiter le passage de fluide entre la bague 50 et le raccord 10. Le cas échéant, la surface externe de la bague 50 peut en outre comprendre une rainure annulaire 52 adaptée pour recevoir un joint torique 53 d'étanchéité, afin d'augmenter encore l'étanchéité du connecteur 1 et d'éviter l'introduction de produits tels que des produits désinfectants.

Le joint torique 53 peut par exemple être réalisé dans un matériau élastomérique, tel que du silicone LSR.

En variante, le joint torique 53 est surmoulé directement sur la bague 50, avec ou sans rainure annulaire 52.

La surface interne de la bague 50, qui est en contact avec la valve 40, est de préférence lisse, et peut avoir subi pour cela un traitement de surface de manière à faciliter le mouvement de la bague 50 le long du corps tubulaire 30. Par exemple, la face interne de la bague 50 peut être siliconée.

Le glissement de la bague 50 est encore amélioré par le fait que la surface externe de la valve 40 et du corps tubulaire 30 est continue et de diamètre constant.

La bague 50 peut par exemple être réalisée dans un matériau plastique du type PBT.

Le connecteur mâle 1 comprend en outre un moyen de rappel 60, adapté pour ramener la bague coulissante 50 dans sa position de repos.

Dans les formes de réalisation illustrées sur les figures, le moyen de rappel 60 est un ressort élastique coaxial au corps tubulaire 30, s'étendant entre la face proximale 54 de la bague coulissante 50 et la face distale du renfoncement radial 33 du corps tubulaire 30. De la sorte, lorsque la bague coulissante 50 est déplacée en direction de sa position de connexion, le ressort 60 exerce sur la bague 50 une force de rappel qui tend à pousser la bague 50 en direction inverse, vers l'extrémité distale du connecteur 1.

Cette forme de réalisation du moyen de rappel 60 n'est cependant pas limitative. Il peut également s'agir d'un soufflet travaillant en compression, etc.

De préférence, lorsque le moyen de rappel 60 est au repos, la bague coulissante 50 se trouve également en position de repos.

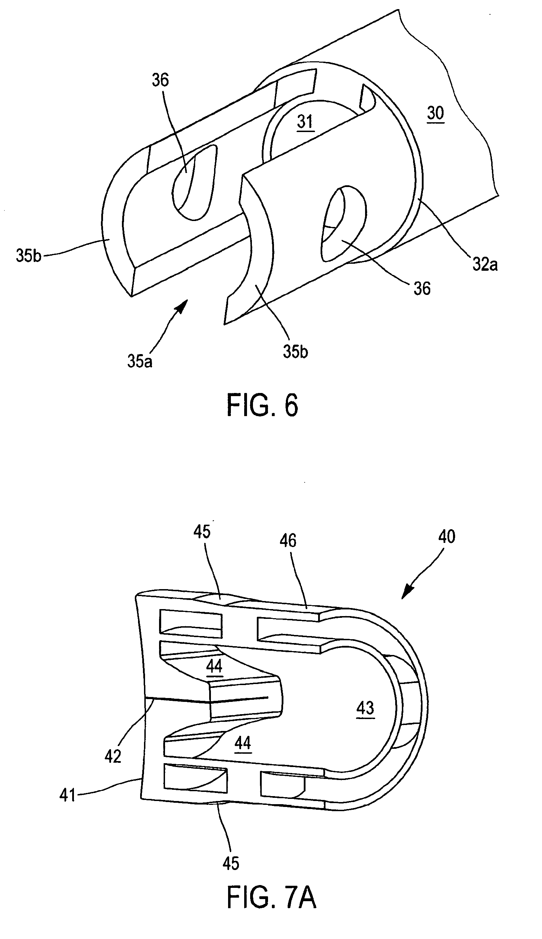

Selon une forme de réalisation illustrée en particulier sur la figure 6, le corps tubulaire 30 peut en outre être fendu à partir de son extrémité distale en direction de son extrémité proximale. De préférence, la fente 35a du corps tubulaire 30 s'étend sur une partie seulement du corps 30, par exemple dans la partie distale qui est en contact avec la valve 40, et confère à la partie distale du corps une certaine élasticité radiale.

Avantageusement, la fente 35a du corps tubulaire 30 est sensiblement parallèle à la fente 42 de la valve 40.

Par ailleurs, selon une forme de réalisation préférée, la fente 35a est réalisée dans le corps tubulaire 30 de sorte que les bords formant ladite fente 35a soient distants l'un de l'autre. La partie distale du corps tubulaire 30 est donc formée de deux pattes latérales 35b séparées par la fente 35a, comme illustré sur la figure 6. Ainsi, lorsque la valve 40 est fixée sur la partie distale du corps tubulaire 30 par surmoulage, ce surmoulage est alors réalisé de sorte que les parois latérales 46 de la valve 40 remplissent la fente 35a et recouvre les pattes 35b du corps tubulaire 30.

Avantageusement, dans la position de repos de la bague coulissante 50, les pattes latérales 35b du corps tubulaire 30 sont contraintes radialement par la bague 50 en direction du canal interne 31 du corps tubulaire 30 afin d'améliorer encore l'étanchéité de la fente 42 de la valve 40, ce qui améliore la tenue en contre-pression de la valve 40.

Par ailleurs, l'espace créé par la fente 35a permet de la réalisation d'une fente 42 de largeur quelconque, sans qu'elle soit limitée par la partie distale du corps tubulaire 30.

Nous verrons dans la suite de la description la fonction de cette fente 35a dans le connecteur mâle 1.

Le raccord distal 10 est fixé sur le corps tubulaire 30 et la bague coulissante 50, de sorte que son extrémité distale 12 vienne en butée et en prise avec le renflement radial 33 et qu'il encapsule la bague coulissante 50 et la valve 40. Le raccord distal 10 est donc immobile en translation par rapport au corps tubulaire 30. Selon la forme de réalisation des moyens d'encliquetage 33a, le raccord distal peut en revanche être mobile en rotation autour du corps tubulaire 30, par exemple lorsque la saillie 33 et la gorge 13 sont annulaires et s'étendent coaxialement au corps tubulaire 30.

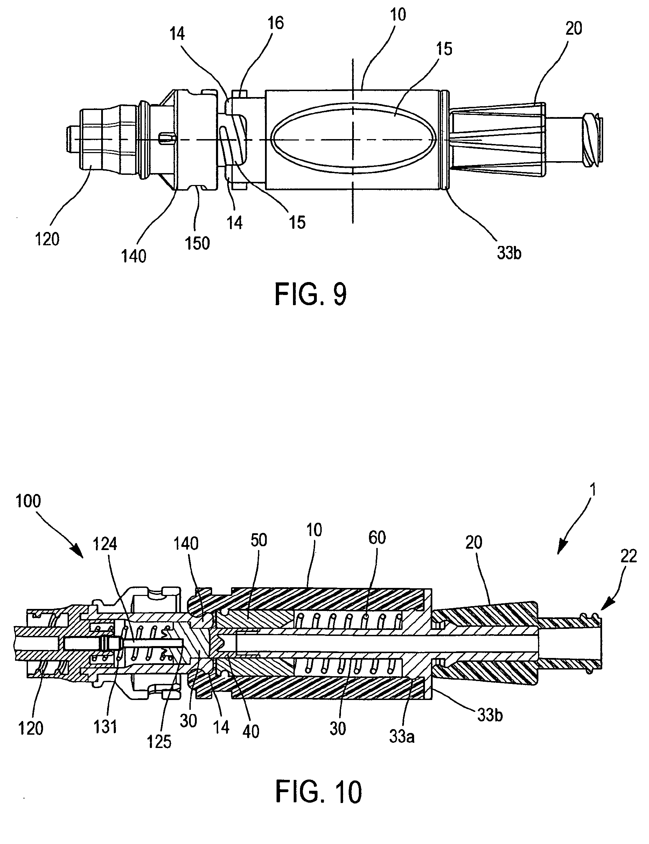

Dans la forme de réalisation illustrée en figures 10, 12 et 13, le raccord distal 10 comprend en outre, au niveau de son extrémité distale 11, au moins une paroi de centrage 14 courbe, qui prolonge la surface externe de l'extrémité distale du raccord 10. Une telle paroi de centrage 14 permet de faciliter l'alignement sur un même axe du connecteur mâle 1 et du connecteur femelle correspondant lors de leur connexion, ce qui garantit leur bon fonctionnement et évite leur dégradation.

De préférence, le raccord distal 10 comprend au moins deux parois de centrage 14 en regard, séparées radialement par deux échancrures 15, de manière à faciliter le nettoyage des surfaces distales affleurantes 41 et 51 de la valve 40 et de la bague coulissante 50.

La face interne des parois de centrage 14 peut en outre comprendre des protubérances inclinées 14a s'étendant radialement vers l'intérieur afin d'améliorer encore le guidage d'un connecteur femelle 100.

L'extrémité distale 11 du raccord distal 10 comprend en outre des moyens de verrouillage 16, de préférence à baïonnette. Ici, les moyens de verrouillage sont deux ergots mâles 16 d'un système à baïonnette, s'étendant chacun depuis une surface externe d'une paroi de centrage 14.

En variante (non illustrée sur les figures), l'extrémité distale 11 du raccord distal 10 comprend une jupette distale, présentant des moyens de verrouillage à baïonnette femelles.

Ce type de moyens de verrouillage 16 a l'avantage, en comparaison avec les moyens de verrouillage conventionnels du type filetage, de présenter des surfaces globalement lisses de sorte qu'elles sont faciles d'accès et donc faciles à nettoyer. Par ailleurs, ils assurent un verrouillage simple et sûr du connecteur mâle 1 avec un connecteur femelle 100 correspondant.

Par ailleurs, le raccord distal 10 comprend en outre des moyens de préhension 15 du connecteur mâle 1. Ici, ils sont au nombre de deux et ont la forme de deux renfoncements qui s'étendent radialement de part et d'autre du raccord distal 10. Ces moyens de manipulation 15 permettent de maintenir fermement le connecteur mâle 1 lors de sa connexion avec un connecteur femelle 100.

Le raccord proximal 20 comporte quant à lui, au niveau de son extrémité proximale 22, une entrée 23 connectable conventionnelle de type « Luer Lock » femelle, apte à être montée sur une seringue (ou tout autre matériel médical adapté) présentant une extrémité de connexion de type « Luer» mâle « Slip » (lisse) ou « Lock » (avec verrouillage), de façon non représentée sur les figures. Cette entrée connectable 23 est de préférence étanche à des pressions allant jusqu'à 3 bars. Pour cela, par exemple, le diamètre externe de la partie proximale 34 du corps tubulaire 30 est légèrement plus grand que le diamètre interne du raccord proximal 20 au niveau de son entrée connectable 23.

Par ailleurs, le raccord proximal 20 comprend un organe d'accrochage 24 cylindrique de révolution coaxial avec le corps tubulaire 30, adapté pour coopérer avec l'organe d'accrochage 34a correspondant de la zone d'accrochage du corps tubulaire 30. De la sorte, le raccord proximal 20 est fixé en translation sur le corps tubulaire 30, tout en restant mobile en rotation autour de l'axe de celui-ci. Tout mouvement de rotation involontaire du connecteur mâle 1 (par exemple lors de la déconnexion du connecteur mâle 1 et d'un connecteur femelle 100 par rotation desdits connecteurs 1 et 100 afin de déconnecter leurs moyens de verrouillage à baïonnettes 16) est donc compensé par la libre rotation du raccord distal 20 par rapport au corps tubulaire 30, évitant ainsi des déconnexions accidentelles du connecteur mâle 1 et de la seringue.

Nous allons à présent décrire un procédé d'utilisation d'un ensemble de connecteurs comprenant un connecteur mâle 1 conforme à l'invention et un connecteur femelle correspondant.

Ici, le connecteur femelle correspond au connecteur 100 décrit précédemment en référence aux figures 1 et 2 annexées et détaillé dans le document EP 2011/05231. Ceci n'est cependant pas limitatif.

Au cours d'une première étape, on nettoie les extrémités distales des deux connecteurs 1 et 100. Ce nettoyage est d'autant plus facile que la valve 40 et la membrane 130 des connecteurs mâle 1 et femelle 100 respectivement affleurent l'extrémité distale des raccords distaux de sorte que la surface distale de chaque connecteur 1, 100 soit lisse. Le cas échéant, le remplacement des filetages conventionnels par les moyens de verrouillage à baïonnette 16, 150 facilite également ce nettoyage, grâce au décalage de la jupette 151 du connecteur femelle 100, qui permet d'accéder facilement aux filets de l'extrémité distale 152 du connecteur 100, ainsi qu'à l'absence de filets conventionnels sur les moyens de verrouillage du connecteur mâle.

Une fois le nettoyage réalisé, l'opérateur met en contact les extrémités distales respectives des connecteurs mâle et femelle 100. Pour cela, il applique la surface concave de la valve 40 sur la surface convexe de la membrane 130 et l'extrémité distale du raccord distal 140 sur l'extrémité distale 51 de la bague coulissante 50, comme cela est illustré sur les figures 9 et 10, puis connecte l'ensemble.

On remarquera que les raccords distaux 140 et 10 des deux connecteurs femelle 100 et mâle 1 sont structurellement similaires (en dehors bien entendu de leurs moyens de verrouillage respectifs) : la membrane 130 et la valve 40 affleurent en effet l'extrémité distale de leur raccord distal 140, 10, de sorte que le couplage/découplage du connecteur mâle 1 et du connecteur femelle 100 n'est réalisé qu'en un seul mouvement axial. Il n'est en effet plus nécessaire de fixer les raccords distaux 140, 10 des connecteurs femelle 100 et mâle 1 avant de commencer à pousser les connecteurs l'un vers l'autre, ces deux opérations étant réalisées simultanément grâce à la structure des raccords distaux 140, 240.

Ici, l'opérateur place les moyens de verrouillage respectifs du connecteur mâle et femelle en regard, puis les verrouille. Dans l'exemple de réalisation illustré, l'opérateur place par exemple les ergots 16 du connecteur mâle 1 en regard des gorges 150 correspondantes du connecteur femelle 100, puis fait glisser les connecteurs 100, 1 relativement l'un par rapport à l'autre en poussant le connecteur mâle 1, en prenant appui sur les moyens de centrage 14 de son raccord distal 10, vers le connecteur femelle 100.

Au cours de cette manipulation, la valve 40 du connecteur mâle 1 pousse sur l'extrémité distale de la membrane 130 du connecteur femelle 100, tandis que le raccord distal 140 du connecteur femelle 100 pousse sur la bague coulissante 50 du connecteur mâle. En conséquence, la valve 40 et une partie du corps tubulaire 30 du connecteur mâle 1 pénètrent dans le raccord distal 140 du connecteur femelle 100, tandis que l'extrémité distale du raccord distal 140 du connecteur mâle 100 pénètre dans l'extrémité distale 11 du raccord distal 10 du connecteur mâle 1.

Pour cela, la valve 40 contraint les moyens élastiques 131 à se comprimer, de sorte que la membrane 30 recule vers sa position de connexion, ce qui force l'extrémité distale 125 du tube creux 124 à traverser, par le biais de la fente 133, le septum situé à l'extrémité distale de la membrane 130. Simultanément, l'extrémité distale du raccord distal 140 du connecteur femelle 100, qui est en appui contre la bague coulissante 50, contraint le moyen de rappel 60 à se comprimer, et déplace ainsi la bague coulissante 50 vers sa position de connexion. La valve 40 et le corps tubulaire 30 en revanche ne se déplacent par rapport au raccord distal 10 et au raccord proximal 20 du connecteur mâle 1.

De plus, la valve 40 ne s'ouvre pas tant que l'extrémité distale 125 du tube creux 124 du connecteur femelle 100 ne traverse pas la membrane, notamment grâce à la rigidité axiale conférée par la partie distale du corps tubulaire 30 sur laquelle est fixée la valve 40.

Selon une forme de réalisation préférée, on choisit le matériau et l'épaisseur de la valve 40 de sorte que celle-ci soit suffisamment résistante lorsqu'elle pousse la membrane 130 pour ne pas s'ouvrir et s'affaisser en direction du corps tubulaire 30. L'objectif en effet est que la valve 40 soit apte à transmettre à la membrane 130 du connecteur femelle 100 la force de poussée appliquée par l'opérateur sur les moyens de préhension 14 pour contraindre la membrane 130 du connecteur femelle 100 à reculer vers sa position de connexion, dans laquelle l'extrémité distale 125 du tube de connexion 124 traverse la membrane 130.

Cette résistance à l'affaissement de la valve 40 est en outre renforcée axialement par le fait que la valve 40 est fixée sur la partie distale du corps tubulaire 30. Au surplus, la présence des pattes latérales 35b renforce encore le cas échéant la rigidité axiale de la valve 40.

Une fois que l'extrémité distale 125 du tube 124 a traversé l'épaisseur de membrane de l'extrémité distale de la membrane 130, cette extrémité distale 125 du tube 124 est en contact d'appui avec la valve 40 du connecteur mâle 1. Etant donné que l'opérateur continue à pousser les connecteurs mâle 1 et femelle 100 l'un vers l'autre, l'extrémité distale 125 du tube 124 appuie sur l'extrémité distale 41 de la valve 40, pour finalement pénétrer dans la fente 42 de la valve 40. En effet, à ce stade, les moyens élastiques sont déjà fortement comprimés et exercent une force de rappel supérieure à celle des bords de la fente 42, de sorte que ceux-ci cèdent et s'écartent pour laisser le passage au tube de connexion 124.

La poussée se poursuit jusqu'à ce que l'extrémité distale 125 du tube 124 du connecteur femelle 100 soit en connexion fluidique avec le corps tubulaire 30 du connecteur mâle 1. Dès lors, la connexion et un passage de fluide sont réalisés entre les connecteurs mâle 1 et femelle 100.

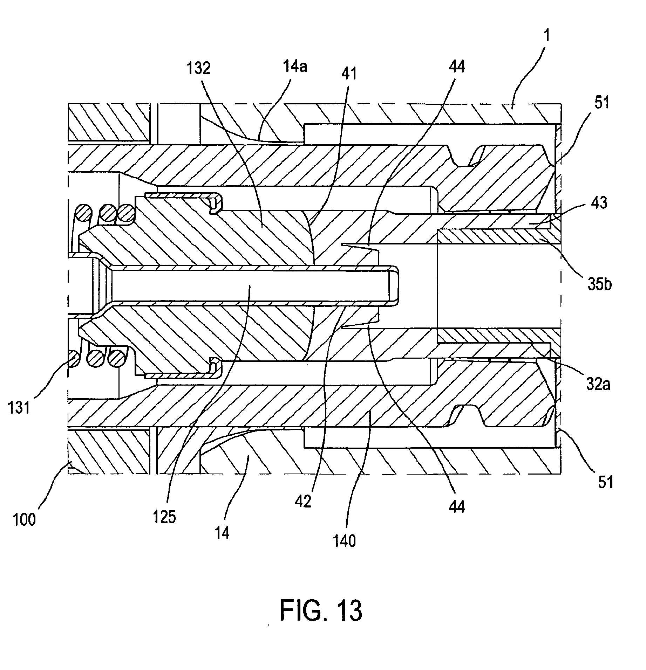

On notera ici que l'extrémité distale 125 du tube de connexion 124 du connecteur femelle 100 ne pénètre pas nécessairement dans l'extrémité distale du corps tubulaire 30. Il suffit en effet qu'elle se trouve adjacente à celui-ci, comme illustré sur les figures 12 et 13.

L'opérateur fait alors tourner les connecteurs l'un par rapport à l'autre afin de positionner les ergots 16 du connecteur mâle en butée dans les gorges correspondantes 150 du connecteur femelle 100, et verrouiller l'ensemble de connecteurs 100, 1.

Afin de faciliter la pénétration de l'extrémité distale 125 du tube de connexion 124 du connecteur femelle 100 dans la fente 42 de la valve 40, le corps tubulaire 30 s'ouvre le long de sa fente 35a. Cette ouverture est facilitée par la présence des pattes latérales 35b, qui confèrent une flexibilité (dans la direction normale au plan de la fente 35a) à l'extrémité distale du corps tubulaire 30 et permettent sa déformation élastique sous l'action du tube de connexion 124 sur la valve 40. De la sorte, lorsque les bords de la fente 42 s'écartent finalement pour laisser le passage à l'extrémité distale 125 du tube de connexion 124, la valve 40 s'affaisse en partie et les pattes latérales 35b du corps tubulaire 30 se déforment élastiquement de manière à s'éloigner l'une de l'autre, réduisant ainsi la résistance axiale qu'elles exercent sur la valve 40 afin de faciliter le passage de l'extrémité distale 125.

On remarquera que, à ce stade de la connexion, la bague coulissante 50 est proche de sa position de connexion, de sorte qu'elle n'empêche pas cette ouverture. En revanche, en position, de repos, la bague coulissante 50 tend à maintenir la fente 35a du corps tubulaire 30 fermée.

Le cas échéant, l'écartement des bords de la fente 42 peut également être facilité par la gorge 44.

A la déconnexion, l'opérateur déverrouille les moyens de verrouillage des connecteurs 1 et 100, par exemple en faisant tourner les connecteurs l'un par rapport à l'autre dans le cas de moyens de verrouillage à baïonnettes, puis écarte les connecteurs 1, 100 avec l'aide de la poussée des moyens élastiques 131 et du moyen de rappel 60. Progressivement, l'extrémité libre 125 du tube de connexion 124 sort de la valve 40, ce qui permet de refermer la fente 42. Cette fermeture de la fente 42 est encore améliorée grâce au retour élastique des pattes latérales 35b, qui tend à ramener les pattes latérales 35b en position initiale de repos, dans laquelle les pattes latérales 35b exercent un effort radial en direction du canal interne 31 sur la fente 42.

La membrane 130 recouvre ensuite progressivement l'extrémité libre 125 du tube 124 jusqu'à l'obturer de manière étanche : la membrane 130 se trouve alors en position de repos.

Parallèlement, la valve 40 et le corps tubulaire 30 sortent du raccord distal 140 du connecteur femelle 100, qui lui-même sort progressivement du raccord distal 10 du connecteur mâle 1, laissant ainsi la bague coulissante 50 revenir dans sa position de repos.

Lors d'une connexion ou d'une déconnexion, l'étanchéité est assurée tout le long de l'opération. En effet, l'étanchéité est assurée par le contact à tout moment de la membrane 130 et de la valve 40.

Par ailleurs, les risques de formation d'une goutte au moment de la déconnexion sont fortement limités, voire supprimés. En effet, la membrane 130 et la valve 40 ne sont plus traversées que par un seul élément, à savoir le tube de connexion 124 qui est au surplus très fin, alors que dans l'art antérieur, le connecteur mâle comprend lui aussi un tube de connexion qui traverse les deux membranes. Le tube de connexion des connecteurs mâles de l'art antérieur est en outre relativement gros, en comparaison avec le tube de connexion 124, de sorte que la fente de la membrane 130 et la fente 42 de la valve 40 doivent davantage s'ouvrir pour le laisser passer.

En utilisant un tube de connexion fin 124 pour le connecteur femelle 100, la fente 133 de la membrane 130 et de la valve 40 s'ouvrent donc beaucoup moins que dans l'art antérieur, de sorte qu'il devient beaucoup plus difficile pour le fluide d'y pénétrer, diminuant ainsi la formation de gouttes au moment de la déconnexion.

Bien entendu, il est possible d'apporter à l'invention de nombreuses modifications sans sortir du cadre de celle-ci.

## Revendications

1. Connecteur mâle (1) pour un circuit liquide, comprenant :
- un raccord distal (10) comprenant une partie tubulaire définissant un passage,
- un raccord proximal (20),
- un organe de connexion (30) s'étendant de manière fixe et coaxiale dans la partie tubulaire du raccord distal (10), ledit organe de connexion étant constitué d'un corps tubulaire (30) sur lequel est fixé, au niveau d'une extrémité libre (32), une valve (40) cylindrique déformable élastiquement et fermée au niveau d'une extrémité distale (41) par une épaisseur de valve traversée par une fente (42),
**caractérisé en ce qu'**il comprend en outre une bague coulissante (50) montée sur le corps tubulaire (30), ladite bague coulissante (30) étant mobile entre une position inactive, dans laquelle elle encapsule les parois (46) de la valve (40) en laissant libre l'extrémité distale (41) de ladite valve (40), et une position de connexion, dans laquelle elle est située à distance de l'extrémité distale (41) de la valve (40) et dévoile tout ou partie des parois latérales (46) de la valve (40).

2. Connecteur mâle (1) selon la revendication 1, comprenant en outre un moyen de rappel (60) prenant appui entre la bague coulissante (50) et le corps tubulaire (30), et adapté pour ramener la bague coulissante (50) vers sa position de repos.

3. Connecteur mâle (1) selon l'une des revendications 1 ou 2, dans lequel la valve (40) présente en outre au moins une gorge borgne (44) débouchant sur le corps tubulaire (30), ladite gorge (44) s'étendant de manière sensiblement parallèlement à la fente (42), coaxialement au corps tubulaire (30), ou autour de la fente (42).

4. Connexion mâle (1) selon l'une des revendications 1 à 3, dans lequel la valve (40) est surmoulée sur le corps tubulaire (30).

5. Connecteur mâle (1) selon l'une des revendications 1 à 4, dans lequel, en position de repos, une surface distale de l'extrémité distale (41) de la valve (40) affleure une surface distale de l'extrémité distale (51) de la bague coulissante (50).

6. Connecteur mâle (1) selon l'une des revendications 1 à 5, dans lequel la valve (40) comprend en outre, au niveau de la fente (42), une surépaisseur radiale (45) s'étendant coaxialement au corps tubulaire (30).

7. Connecteur mâle (1) selon la revendication 6, dans lequel la surépaisseur (45) a la forme d'une ellipse dont un petit diamètre est sensiblement égal à un diamètre interne de la bague coulissante (50) et un grand diamètre mesure quelques dixièmes de millimètres de plus que le petit diamètre.

8. Connecteur mâle (1) selon l'une des revendications 1 à 7, dans lequel le raccord distal (10) encapsule le corps tubulaire (30) et la bague coulissante (50) de manière à ne laisser libre que leurs extrémités distales (41, 51) respectives.

9. Connecteur mâle (1) selon la revendication 8, dans lequel la bague coulissante (50) comprend en outre un joint d'étanchéité (53) s'étendant entre la bague coulissante (50) et le raccord distal (10).

10. Connecteur mâle (1) selon l'une des revendications 1 à 9, dans lequel le corps tubulaire (30) présente en outre une fente (35a) s'étendant parallèlement à la fente (42) de la valve (40) depuis son extrémité distale (32) en direction de son extrémité proximale (34), de manière à définir deux pattes latérales (35b).

11. Procédé de fabrication d'un connecteur mâle (1) selon l'une des revendications 1 à 10, comprenant les étapes consistant à :
- prévoir un corps tubulaire (30) ;
- fixer la valve (40) sur une extrémité distale (32) du corps tubulaire (30) ; et
- fixer le corps tubulaire (30) sur le raccord proximal (20) ;
**caractérisé en ce qu'**il comprend en outre les étapes consistant à :
- monter la bague coulissante (50) sur l'extrémité distale (32) du corps tubulaire (30) ; et
- fixer le raccord distal (10) sur le corps tubulaire (30).

12. Procédé de fabrication selon la revendication 11, dans lequel la valve (40) est fixée par surmoulage sur le corps tubulaire (30).

13. Ensemble de connexion pour un circuit liquide, comprenant un connecteur mâle (1) selon l'une des revendications 1 à 12, et un connecteur femelle (100), ledit connecteur femelle (100) comprenant :
- un raccord distal (140), et
- un raccord proximal (120), le raccord distal (140) comprenant une partie tubulaire, dans laquelle s'étend de manière coaxiale un organe de connexion (124) monté fixe sur le raccord proximal (120), et une membrane cylindrique déformable élastiquement (130) fermée au niveau d'une extrémité distale (132) par un septum traversé par une fente (133), et mobile entre une position d'obturation, dans laquelle la membrane (130) recouvre une extrémité libre (125) de l'organe de connexion (124) de manière étanche, et une position de connexion, dans laquelle la membrane (130) et la valve (40) sont traversées par l'organe de connexion (124).

14. Ensemble de connexion selon la revendication 13, dans lequel la forme et les dimensions de la valve (40) du connecteur mâle sont sensiblement égales à la forme et aux dimensions de la membrane (130), de sorte que lors de la connexion du connecteur mâle (1) avec le connecteur femelle (100), la valve (40) est apte à pénétrer dans l'extrémité distale (132) du raccord distale (140) du connecteur femelle (100).

15. Ensemble de connexion selon l'une des revendications 13 ou 14, dans lequel le connecteur femelle (100) et le connecteur mâle (1) comprennent chacun des moyens de verrouillage complémentaires à baïonnettes (151, 16).

16. Ensemble de connexion selon l'une des revendications 13 à 15, dans lequel les dimensions et la forme du raccord distal (140) du connecteur femelle (100) sont sensiblement identiques aux dimensions et à la forme de la bague coulissante (50), de sorte que lors de la connexion du connecteur mâle (1) avec le connecteur femelle (100), l'extrémité distale (140) du connecteur femelle (100) soit apte à pénétrer dans l'extrémité distale (11) du raccord distal (10) du connecteur mâle (1).

17. Ensemble de connexion selon l'une des revendications 13 à 16, dans lequel le corps tubulaire (30) du connecteur mâle (1) présente en outre une fente (35a) s'étendant parallèlement à la fente (42) de la valve (40) depuis son extrémité distale (32) en direction de son extrémité proximale (34) de manière à définir deux pattes latérales (35b), et dans lequel les pattes latérales (35b) du connecteur mâle (1) se déforment élastiquement lors de la pénétration de l'organe de connexion (124) du connecteur femelle (100) dans la fente (42) de la valve (40) du connecteur mâle (1).

## Patentansprüche

1. Kontaktstift (1) für einen Flüssigkeitskreislauf, umfassend:
- ein distales Verbindungsstück (10), welches einen röhrenförmigen Abschnitt umfasst, der einen Durchgang definiert,
- ein proximales Verbindungsstück (20),
- ein Anschlussteil (30), das sich feststehend und koaxial in dem röhrenförmigen Abschnitt des distalen Verbindungsstücks (10) erstreckt, wobei das Anschlussteil aus einem röhrenförmigen Körper (30) besteht, an dem auf Höhe eines freien Endes (32) ein elastisch verformbares zylindrisches Ventil (40) befestigt ist, welches auf Höhe eines distalen Endes (41) durch eine Ventildicke, die von einem Schlitz (42) durchlaufen wird, verschlossen wird,
**dadurch gekennzeichnet, dass** er außerdem einen verschiebbaren Ring (50) umfasst, der auf dem röhrenförmigen Körper (30) angebracht ist, wobei der verschiebbare Ring (50) zwischen einer inaktiven Position, in der er die Wände (46) des Ventils (40) einkapselt, wobei er das distale Ende (41) des Ventils (40) frei lässt, und einer Verbindungsposition, in der er sich im Abstand zu dem distalen Ende (41) des Ventils (40) befindet und die Seitenwände (46) des Ventils (40) teilweise oder vollständig freilegt, beweglich ist.

2. Kontaktstift (1) gemäß Anspruch 1, welcher außerdem ein Rückholmittel (60) umfasst, welches zwischen dem verschiebbaren Ring (50) und dem röhrenförmigen Körper (30) gehalten wird und geeignet ist, den verschiebbaren Ring (50) in seine Ruheposition zurückzubringen.

3. Kontaktstift (1) gemäß einem der Ansprüche 1 oder 2, bei dem das Ventil (40) außerdem wenigstens eine Blindvertiefung (44) aufweist, die auf dem röhrenförmigen Körper (30) mündet, wobei sich die Vertiefung (44) in etwa parallel zu dem Schlitz (42), koaxial zu dem röhrenförmigen Körper (30) oder um den Schlitz (42) herum erstreckt.

4. Kontaktstift (1) gemäß einem der Ansprüche 1 bis 3, bei dem das Ventil (40) auf den röhrenförmigen Körper (30) aufgeformt ist.

5. Kontaktstift (1) gemäß einem der Ansprüche 1 bis 4, bei dem im Ruhezustand eine distale Oberfläche des distalen Endes (41) des Ventils (40) mit einer distalen Oberfläche des distalen Endes (51) des verschiebbaren Rings (50) fluchtet.

6. Kontaktstift (1) gemäß einem der Ansprüche 1 bis 5, bei dem das Ventil (40) außerdem auf Höhe des Schlitzes (42) eine radiale Überdicke (45) aufweist, die sich koaxial zu dem röhrenförmigen Körper (30) erstreckt.

7. Kontaktstift (1) gemäß Anspruch 6, bei dem die Überdicke (45) die Form einer Ellipse hat, deren kleiner Durchmesser in etwa einem inneren Durchmesser des verschiebbaren Rings (50) entspricht und deren großer Durchmesser einige Zehntel Millimeter über dem kleinen Durchmesser misst.

8. Kontaktstift (1) gemäß einem der Ansprüche 1 bis 7, bei dem das distale Verbindungsstück (10) den röhrenförmigen Körper (30) und den verschiebbaren Ring (50) derart einkapselt, dass lediglich ihre jeweiligen distalen Enden (41, 51) freigelassen werden.

9. Kontaktstift (1) gemäß Anspruch 8, bei dem der verschiebbare Ring (50) außerdem ein Dichtungsgelenk (53) aufweist, das sich zwischen dem verschiebbaren Ring (50) und dem distalen Verbindungsstück (10) erstreckt.

10. Kontaktstift (1) gemäß einem der Ansprüche 1 bis 9, bei dem der röhrenförmige Körper (30) außerdem einen Schlitz (35a) aufweist, der sich parallel zu dem Schlitz (42) des Ventils (40) von seinem distalen Ende (32) in Richtung seines proximalen Endes (34) erstreckt, derart, dass zwei laterale Lappen (35b) definiert werden.

11. Verfahren zur Herstellung eines Kontaktstifts (1) gemäß einem der Ansprüche 1 bis 10, welches die folgenden Schritte umfasst:
- Bereitstellen eines röhrenförmigen Körpers (30);
- Befestigen des Ventils (40) an einem distalen Ende (32) des röhrenförmigen Körpers (30); und
- Befestigen des röhrenförmigen Körpers (30) an dem proximalen Verbindungsstück (20);
**dadurch gekennzeichnet, dass** es außerdem die folgenden Schritte umfasst:
- Anbringen des verschiebbaren Rings (50) an dem distalen Ende (32) des röhrenförmigen Körpers (30); und
- Befestigen des distalen Verbindungsstücks (10) an dem röhrenförmigen Körper (30).

12. Herstellungsverfahren gemäß Anspruch 11, bei dem das Ventil (40) durch Aufformen auf dem röhrenförmigen Körper (30) befestigt wird.

13. Verbindungseinheit für einen Flüssigkeitskreislauf, umfassend einen Kontaktstift (1) gemäß einem der Ansprüche 1 bis 12 und eine Kontakthülse (100), wobei die Kontakthülse (100) umfasst:
- ein distales Verbindungsstück (140) und
- ein proximales Verbindungsstück (120), wobei das distale Verbindungsstück (140) umfasst: einen röhrenförmigen Abschnitt, in dem sich koaxial ein Anschlussteil (124) erstreckt, das fest an dem proximalen Verbindungsstück (120) angebracht ist, und eine elastisch verformbare zylindrische Membran (130), die auf Höhe eines distalen Endes (132) durch ein Septum verschlossen wird, das von einem Schlitz (133) durchsetzt wird, und zwischen einer Verschlussposition, in der die Membran (130) ein freies Ende (125) des Anschlussteils (124) feuchtigkeitssicher abdeckt, und einer Anschlussposition, in der die Membran (130) und das Ventil (40) von dem Anschlussteil (124) durchsetzt werden, beweglich ist.

14. Verbindungseinheit gemäß Anspruch 13, bei der die Form und die Abmessungen des Ventils (40) des Kontaktstifts in etwa der Form und den Abmessungen der Membran (130) entsprechen, derart, dass bei Verbindung des Kontaktstifts (1) mit der Kontakthülse (100) das Ventil (40) imstande ist, in das distale Ende (132) des distalen Verbindungsstücks (140) der Kontakthülse (100) einzudringen.

15. Verbindungseinheit gemäß einem der Ansprüche 13 oder 14, bei der die Kontakthülse (100) und der Kontaktstift (1) jeweils Mittel zur komplementären Bajonettverriegelung (151, 16) umfassen.

16. Verbindungseinheit gemäß einem der Ansprüche 13 bis 15, bei der die Abmessungen und die Form des distalen Verbindungsstücks (140) der Kontakthülse (100) in etwa identisch mit den Abmessungen und der Form des verschiebbaren Rings (50) sind, derart, dass bei der Verbindung des Kontaktstifts (1) mit der Kontakthülse (100) das distale Ende (140) der Kontakthülse (100) imstande ist, in das distale Ende (11) des distalen Verbindungsstücks (10) des Kontaktstifts (1) einzudringen.

17. Verbindungseinheit gemäß einem der Ansprüche 13 bis 16, bei der der röhrenförmige Körper (30) des Kontaktstifts (1) außerdem einen Schlitz (35a) aufweist, der sich parallel zu dem Schlitz (42) des Ventils (40) von seinem distalen Ende (32) in Richtung seines proximalen Endes (34) erstreckt, derart, dass zwei laterale Lappen (35b) definiert werden, und bei der sich die lateralen Lappen (35b) des Kontaktstifts (1) bei Eindringen des Anschlussteils (124) der Kontakthülse (100) in den Schlitz (42) des Ventils (40) des Kontaktstifts (1) elastisch verformen.

## Claims

1. A male connector (1) for a liquid circuit, including:
- a distal hub (10) including a tubular portion defining a passage,
- a proximal hub (20),
- a connection member (30) extending fixedly and coaxially within the tubular portion of the distal hub (10), said connection member consisting of a tubular body (30) whereon is fastened, at a free end (32), a cylindrical, elastically deformable valve (40) closed at a distal end (41) by a thickness of valve through which passes a slit (42),
**characterized in that** it also includes a sliding ring (50) mounted on the tubular body (30), said sliding ring (50) being movable between an inactive position, wherein it encapsulates the walls (46) of the valve (40) while leaving free the distal end (41) of said valve (40), and a connection position wherein it is situated at a distance from the distal end (41) of the valve (40) and exposes all or a portion of the sidewalls (46) of the valve (40).

2. The male connector (1) according to Claim 1, also including a biasing means (60) supported between the sliding ring (50) and the tubular body (30) and designed to bias the sliding ring (50) toward its rest position.

3. The male connector (1) according to one of Claims 1 or 2, wherein the valve (40) also has at least one blind groove (44) leading to the tubular body (30), said groove (44) extending substantially parallel to the slit (42), coaxially with the tubular body (30), or around the slit (42).

4. The male connector (1) according to one of Claims 1 to 3, wherein the valve (40) is overmolded onto the tubular body (30).

5. The male connector (1) according to one of Claims 1 to 4, wherein, in the rest position, a distal surface of the distal end (41) of the valve (40) is flush with a distal surface of the distal end (51) of the sliding ring (50).

6. The male connector (1) according to one of Claims 1 to 5, wherein the valve (40) also includes, at the slit (42), a radial thickenied portion (45) extending coaxially to the tubular body (30).

7. The male connector (1) according to Claim 6, wherein the thickened portion (45) has a shape of an ellipse, a minor diameter whereof is substantially equal to an inner diameter of the sliding ring (50) and a major diameter whereof measures a few tenths of a millimeter more than the minor diameter.

8. The male connector (1) according to one of Claims 1 to 7, wherein the distal hub (10) encapsulates the tubular body (30) and the sliding ring (50) so as to leave only their respective distal ends (41, 51) free.

9. The male connector (1) according to Claim 8, wherein the sliding ring (50) also includes a seal (53) extending between the sliding ring (50) and the distal hub (10).

10. The male connector (1) according to one of Claims 1 to 9, wherein the tubular body (30) also has a slit (35a) extending parallel to the slit (42) of the valve (40) from its distal ends (32) toward its proximal end (34), so as to define two lateral legs (35b).

11. A method for manufacturing a male connector (1) according to one of Claims 1 to 10, including the steps consisting of:
- providing a tubular body (30);
- fastening the valve (40) onto a distal end (32) of the tubular body (30); and
- fastening the tubular body (30) onto the proximal hub (20);
**characterized in that** it also includes the steps consisting of:
- mounting the sliding ring (50) on the distal end (32) of the tubular body (30); and
- fastening the distal hub (10) onto the tubular body (30).

12. The manufacturing method according to Claim 11, wherein the valve (40) is fastened by overmolding onto the tubular body (30).

13. A connection assembly for a liquid circuit, including a male connector (1) according to one of Claims 1 to 12, and a female connector (100), said female connector (100) including:
- a distal hub (140), and
- a proximal hub (120), the distal hub (140) including a tubular portion wherein extends coaxially a connection member (124) mounted fixedly on the proximal hub (120) and an elastically deformable cylindrical membrane (130) closed at a distal end (132) by a septum through which passes a slit (133), movable between a plugging position wherein the membrane (130) sealingly covers a free end (125) of the connection member (124), and a connection position wherein the connection member (124) passes through the membrane (130) and the valve (40).

14. The connection assembly according to Claim 13, wherein a shape and dimensions of the valve (40) of the male connector are substantially equal to a shape and to dimensions of the membrane (130), so that when connecting the male connector (1) with the female connector (100), the valve (40) is able to penetrate into the distal end (132) of the distal hub (140) of the female connector (100).

15. A connection assembly according to one of Claims 13 or 14, wherein the female connector (100) and the male connector (1) each include complementary bayonet locking means (151, 16).

16. The connection assembly according to one of claims 13 to 15, wherein the dimensions and the shape of the distal hub (140) of the female connector (100) are substantially identical to the dimensions and to the shape of the sliding ring (50), such that during connection of the male connector (1) with the female connector (100), the distal end (140) of the female connector (100) is able to penetrate into the distal end (11) of the distal hub (10) of the male connector (1).

17. The connection assembly according to one of Claims 13 to 16, wherein the tubular body (30) of the male connector (1) also has a slit (35a) extending parallel to the slit (42) of the valve (40) from its distal end (32) toward it proximal end (34) so as to define two lateral legs (35b), and wherein the lateral legs (35b) of the male connector (1) deform elastically during penetration of the connection member (124) of the female connector (100) into the slit (42) of the valve (40) of the male connector (1).
